# EUROPÄISCHE PATENTSCHRIFT

(11) **EP 1 200 018 B1**
(45) Veröffentlichungstag und Bekanntmachung des Hinweises auf die Patenterteilung: **06.05.2004**
(21) Anmeldenummer: 00958323.8
(22) Anmeldetag: 29.07.2000
(51) Int. Cl.: A61F 2/06, A61M 25/00

(54) **EINFÜHRKATHETER FÜR GEFÄSSPROTHESEN**
INSERTION CATHETER FOR VASCULAR PROSTHESES
CATHETER D'INSERTION POUR PROTHESES VASCULAIRES

(30) Priorität: 05.08.1999 DE 19936980
(43) Veröffentlichungstag der Anmeldung: 02.05.2002
(73) Patentinhaber: Aesculap AG & Co. KG, 78532 Tuttlingen (DE)
(72) Erfinder: DWORSCHAK, Manfred, D-78589 Dürbheim (DE); LUTZE, Theodor, D-78582 Balgheim (DE); WEIK, Thomas, D-78532 Tuttlingen (DE); WEISSHAUPT, Dieter, D-78194 Immendingen (DE); ALLENBERG, Jens-Rainer, D-69120 Heidelberg (DE)
(74) Vertreter: Böhme, Ulrich, Dr. Dipl.-Phys.
(86) Internationale Anmeldenummer: PCT/EP2000/007354
(87) Internationale Veröffentlichungsnummer: WO 2001/010345

(56) Entgegenhaltungen:
- EP-A- 0 554 579
- WO-A-98/09583
- WO-A-98/20812
- DE-A- 19 713 280
- US-A- 5 683 451
- US-A- 5 879 380

## Beschreibung

Die Erfindung betrifft einen Einführkatheter, der eine stützstrukturlose Gefäßprothese trägt, zum Einführen der Gefäßprothese in ein Gefäß mit einer langgestreckten, im wesentlichen rohrförmigen Katheterhülle, die ein proximales und ein distales Ende aufweist, wobei an dem distalen Ende eine Austrittsöffnung für die Gefäßprothese vorgesehen ist, wobei die von der Austrittsöffnung definierte Querschnittsfläche kleiner als die Querschnittsfläche der in das Gefäß eingebrachten Gefäßprothese im geöffneten ungedehnten Zustand ist.

Zur Behandlung von Gefäßverschlüssen beim Menschen, insbesondere im peripheren Bereich oder auch zur Ausschaltung abdominaler und thorakaler Aortenaneurysmen, werden derzeit Gefäßstützen eingesetzt, sogenannte "Stents" oder auch "Stent Grafts", die metallische Stützstrukturen aufweisen, welche auch mit Gewebe umhüllt sein können. Diese Stützstrukturen werden mittels eines Katheters auf endoluminalem Weg in den betroffenen Gefäßabschnitt eingebracht.

Die "Stents" oder "Stent Grafts" sind in einem komprimierten, nicht expandierten Zustand auf dem Katheter montiert und können von einem schützenden Hüllrohr umgeben sein, das vor dem Einbringen des Implantats zurückgezogen wird und letzteres freigibt. Die Gefäßstütze nimmt ihre endgültige Form entweder durch Selbstexpansion aufgrund ihrer plastischen Stützstruktur ein oder dadurch, daß die Gefäßstütze auf einen Ballon montiert ist, der durch eine erzwungene Expansion die Gefäßstütze in ihre geöffnete Form überführt.

Derartige Stützstrukturen bergen den Nachteil, daß aufgrund einer Wechselwirkung zwischen dem Metallgerüst und der Gewebehülle unter dynamischer Beanspruchung die Hülle verletzt werden kann und auf diese Weise Endoleckagen entstehen.

Um einer solchen Abnutzung vorzubeugen, können Gefäßprothesen auch ohne metallische oder sonstige Stützstrukturen verwendet werden. Diese haben zusätzlich den Vorteil, daß in Gefäßen mit niedrigen Durchflußraten keine zusätzlichen Hindernisse in Form von Stützstrukturen einwirken, die zu Okklusionen führen können. Insbesondere dünnwandige Prothesen werden vorteilhafterweise verwendet und mit einem Ballon an die präparierte Gefäßwand anmoduliert. Bei derartigen stützstrukturlosen Gefäßprothesen besteht jedoch die Gefahr, beim Einsetzen derselben einen Teilbereich zu verdrehen, woraus letztendlich ein Verschluß der Gefäßprothese resultiert. Mit Einführkathetern der eingangs beschriebenen Art ist ein sicheres und definiertes Einbringen solcher Gefäßprothesen nicht vollständig gewährleistet.

Ein Beispiel einer eingangs beschriebenen Gefäßprothese offenbart die US-A-5,879,380 in Form eines Einführkatheters. Er trägt eine stützstrukturlose Gefäßprothese und dient zum Einführen derselben in ein Gefäß. Er umfaßt eine langgestreckte, im wesentlichen rohrförmige Katheterhülle, die an einem distalen Ende eine Austrittsöffnung für die Gefäßprothese aufweist. Die von der Austrittsöffnung definierte Querschnittsfläche ist kleiner als die Querschnittsfläche der Gefäßprothese in geöffnetem ungedehntem Zustand. Weitere Beispiele für derartige Einführkatheter finden sich in der US-A-5,683,451 sowie der WO 98/20812.

Es ist dementsprechend Aufgabe der vorliegenden Erfindung, einen Einführkatheter der eingangs beschriebenen Art derart auszubilden, daß eine Gefäßprothese besonders einfach und sicher in ein Gefäß des menschlichen Körpers eingeführt werden kann, ohne daß es zu einem Verschluß der Gefäßprothese, beispielsweise durch Verdrehen kommt.

Diese Aufgabe wird erfindungsgemäß dadurch gelöst, daß die Austrittsöffnung eine im Querschnitt von einer Kreisform abweichende Form aufweist und daß sich im Innern der Katheterhülle eine Vorrichtung zum Falten der Gefäßprothese in einem an die Austrittsöffnung angrenzenden Abschnitt erstreckt, wodurch die Gefäßprothese beim Durchtritt durch die Austrittsöffnung eine dafür notwendige Querschnittsform einnimmt.

Bei herkömmlichen Kathetern ist die Austrittsöffnung im Querschnitt kreisförmig. Dadurch ist keine gezielte Führung der Gefäßprothese beim Einsetzen möglich, wenn diese durch Zurückziehen der Katheterhülle von dem Katheter freigegeben wird. Durch die von der Kreisform abweichende Form der Austrittsöffnung werden, zunächst völlig unabhängig von der Querschnittsform an sich, Vorzugsrichtungen definiert, die eine gezielte Führung der Gefäßprothese beim Zurückziehen der Katheterhülle definieren. Die Austrittsöffnung entspricht demnach dem Umfang eines durch die Kreisform definierten Kreises, kann aber grundsätzlich jeden von der Kreisform abweichenden anderen Querschnitt aufweisen. Dadurch ist die Lage des Katheters, insbesondere eine Rotation desselben, jederzeit festzustellen, und es kann einem unbeabsichtigten Verdrehen der Gefäßprothese jederzeit entgegengewirkt werden. Diese gezielte Rotation kann bei geeigneter Wahl der Form der Austrittsöffnung sogar völlig unnötig sein, denn die spezielle Form verhindert durch die dadurch gebildete Führung schon grundsätzlich eine Rotation der Gefäßprothese. Eine Vorrichtung zum Falten der Gefäßprothese erlaubt es, die Prothese auf nahezu beliebige Weise innerhalb der Katheterhülle anzuordnen und sie erst vor dem Austreten aus der Austrittsöffnung in die dafür erforderliche Form zu bringen, nämlich so zu falten, daß die dann von der Prothese eingenommene Querschnittsfläche kleiner ist als die der Austrittsöffnung. Dies erspart insbesondere bei der Vorbereitung des Katheters Zeit, also beim Einführen der Prothese in denselben und erfordert weniger Sorgfalt bei der Vorbereitung.

Besonders vorteilhaft kann es sein, wenn die Außenkontur der Austrittsöffnung eine Umfangslänge aufweist, die mindestens dem Umfang der in das Gefäß eingebrachten Gefäßprothese entspricht. Diese Ausgestaltung ermöglicht es, daß die Gefäßprothese mit ihrer äußeren Oberfläche vollständig an der inneren Wandung der Austrittsöffnung anliegt. Die Gefäßprothese wird daher beim Herausgleiten aus der Katheterhülle durch die Austrittsöffnung über deren gesamten Umfang definiert geführt und ein Verdrehen der Prothese verhindert.

Bei einer anderen bevorzugten Ausführungsform kann vorgesehen sein, daß die Außenkontur der Austrittsöffnung eine Umfangslänge aufweist, die kleiner als der äußere Umfang der in das Gefäß eingebrachten Gefäßprothese ist. Eine solche Austrittsöffnung ermöglicht das Herausgleiten der Prothese aus dem Katheter nur dann, wenn die Prothese gefaltet ist. Durch die unrunde Form wird die gefaltete Prothese dennoch jederzeit verdrehsicher geführt, wobei eine gefaltete Prothese besonders kompakt innerhalb des Katheters angeordnet werden kann. Dies ist insbesondere bei Gefäßen mit sehr geringem Durchmesser von Vorteil.

Günstig ist es dabei, wenn die Austrittsöffnung eine Rechteckform aufweist, die eine kürzere Seitenkante mit einer Breite aufweist, die mindestens einem geradzahligen Vielfachen einer Wandstärke der Gefäßprothese entspricht. Durch die Rechteckform werden zwei jeweils gegenüberliegende, unterschiedlich lange Kanten der Austrittsöffnung vorgegeben, mit denen eine definierte Führung einer gefalteten Prothese besonders leicht möglich ist. Durch die Wahl der Breite des Rechtecks bezogen auf die Wandstärke der Prothese können nur auf eine ganz bestimmte Art und Weise gefaltete Prothesen überhaupt durch die Austrittsöffnung austreten. Dies ermöglicht eine zusätzliche Verdrehsicherung, indem lediglich verdrehsicher gefaltete Prothesen in das Gefäß eingeführt werden können.

Vorteilhafterweise kann vorgesehen sein, daß die Querschnittsfläche der Austrittsfläche im wesentlichen identisch zu der Querschnittsfläche einer auf ihren kleinstmöglichen Querschnitt gefalteten und durch die Austrittsfläche auszubringenden Gefäßprothese ist. Eine derartige Austrittsöffnung ermöglicht eine definierte allseitige Führung der gefalteten Prothese, denn die Austrittsöffnung steht über ihren gesamten Umfang in Kontakt mit nach außen weisenden Teilen der Prothese.

Besonders vorteilhaft ist es, wenn das distale Ende einen Führungskörper umfaßt. Der Führungskörper erleichtert das Einführen des Katheters in das Gefäß und schützt durch seine Anordnung am distalen Ende die Austrittsöffnung beim Einführen in das Gefäß.

Es wäre denkbar, daß der Führungskörper relativ zur Katheterhülle in eine beliebige Richtung bewegbar ist, beispielsweise verschwenkbar, vorteilhafterweise ist jedoch vorgesehen, daß der Führungskörper relativ zur Katheterhülle in einer von der Katheterhülle vorgegebenen Längsrichtung verschiebbar ist. Dies ermöglicht es, den Abstand zwischen dem Führungskörper und der Austrittsöffnung beliebig zu variieren, was beispielsweise dann sinnvoll ist, wenn der Führungskörper dazu dient, die Prothese an der Gefäßwand zu halten.

Grundsätzlich gibt es verschiedene Möglichkeiten, den Führungskörper anzuordnen, beispielsweise an der Katheterhülle, besonders vorteilhaft ist es jedoch, wenn der Führungskörper am Ende eines die Katheterhülle durchsetzenden Führungsstabes angeordnet ist. Der Führungskörper kann mittels des Führungsstabes unabhängig von der Katheterhülle bewegt werden.

Zum Halten der Prothese ist es günstig, einen Haltekörper vorzusehen. Dieser kann an der Katheterhülle oder dem Führungskörper angeordnet sein, besonders vorteilhaft ist es jedoch, wenn am Führungsstab im Bereich des Führungskörpers mindestens ein in seinem äußeren Umfang in radialer Richtung veränderbarer Haltekörper angeordnet ist. Mit diesem läßt sich die Prothese an der Innenwand des Gefäßes vorfixieren, bis sie mit diesem endgültig verbunden wird, beispielsweise durch eine Anastomose.

Vorteilhafterweise kann vorgesehen sein, daß an der Gefäßprothese im Bereich des Führungskörpers mindestens ein in seinem äußeren Umfang in radialer Richtung veränderbarer Haltekörper angeordnet ist. Ein solcher Haltekörper, insbesondere wenn er an einer inneren Umfangswandung der Gefäßprothese angeordnet ist, kann, wenn er seinen äußeren Umfang ändert, vorzugsweise vergrößert, die Gefäßprothese gegen eine innere Umfangswandung des Gefäßes drücken und auf diese Weise die Gefäßprothese an dem Gefäß wenigstens vorübergehend fixieren.

Bei einer vorteilhaften Ausführungsform kann vorgesehen sein, daß der Haltekörper durch eine selbstexpandierbare Gefäßstütze gebildet wird. Eine solche, beispielsweise auch als Stent bezeichnete Gefäßstütze kann ihren äußeren Umfang vergrößern, beispielsweise indem ein den Stent bildendes Gewebe sich entfaltet oder eine zusammengedrückte Schraubenfeder sich ausdehnt.

Günstig ist es, wenn die Gefäßstütze durch ein Metall gebildet wird. Eine solche Gefäßstütze ist besonders stabil und kann darüber hinaus bei Verwendung besonderer Legierungen einen Memory-Effekt aufweisen, so daß die Gefäßstütze ihren äußeren Umfang auch durch Temperaturänderung verändern kann.

Besonders günstig ist es, wenn der Haltekörper mittels eines Fluids inflatierbar ist. Durch eine derartige Konstruktion kann auf aufwendige mechanische Konstruktionen verzichtet werden, beispielsweise auf radial abstehende und in ihrer Länge veränderbare Dorne.

Vorzugsweise kann vorgesehen sein, daß der Haltekörper einen Ballon umfaßt. Ein Ballon nimmt im deflatierten Zustand besonders wenig Raum ein, im inflatierten Zustand kann er praktisch jede beliebige Form einnehmen. Außerdem ist ein Ballon besonders atraumatisch.

Vorteilhafterweise ist vorgesehen, daß im Inneren des Führungsstabes eine mit dem Innern des Haltekörpers in Fluidverbindung stehende Fluidleitung verläuft. Auf diese Weise wird eine separate Führungsleitung außerhalb der Katheterhülle unnötig. Der Führungsstab übt somit eine Doppelfunktion aus, wodurch die Zahl der Teile des Katheters reduziert wird.

Grundsätzlich wäre es denkbar, den Haltekörper einseitig am Führungsstab anzuordnen. Besonders vorteilhaft ist es aber, wenn der Haltekörper den Führungsstab ringförmig umgibt, denn die Prothese kann so gleichmäßig und symmetrisch an die Innenwand des Gefäßes gedrückt werden.

Für die einander zugewandten Enden der Katheterhülle und des Führungskörpers sind prinzipiell alle denkbaren Formen möglich. Gemäß einer bevorzugten Ausführungsform der Erfindung ist jedoch vorgesehen, daß das distale Ende der Katheterhülle und ein proximales Ende des Führungskörpers eine zueinander komplementäre Form aufweisen.

Der Führungskörper und die Katheterhülle können dadurch eine kompakte Form bilden, was insbesondere beim Einführen des Katheters in das Gefäß Vorteile bietet, denn vom Katheter abstehende Teile können das Gefäß ungewollt verletzen.

Dabei ist es besonders günstig, wenn das distale Ende der Katheterhülle durch einen Außenkonus und das proximale Ende des Führungskörpers durch einen Innenkonus gebildet werden. Wird der Führungskörper von der Austrittsöffnung des Katheters wegbewegt, dann bildet das distale Ende der Katheterhülle immer noch ein im wesentlichen stumpfes Ende, durch das das Gefäß normalerweise nicht verletzt werden kann.

Obwohl der Führungskörper auch ein eckiges distales Ende aufweisen könnte, ist es besonders vorteilhaft, wenn das distale Ende des Führungskörpers eine abgerundete Spitze aufweist. Dies reduziert die Verletzungsgefahr für das Gefäß beim Einführen des Katheters.

Der Führungskörper und die Katheterhülle könnten relativ zueinander grundsätzlich in jeder Stellung beabstandet sein, vorteilhafterweise ist jedoch vorgesehen, daß das distale Ende der Katheterhülle durch den Führungskörper verschließbar ist. Auf diese Weise ist es möglich, einen an seinem distalen Ende vollständig geschlossenen Katheter bereitzustellen, der überdies eine völlig glatte Außenhaut aufweisen kann.

Gemäß einer bevorzugten Ausführungsform der Erfindung kann vorgesehen sein, daß an dem distalen Ende der Katheterhülle mindestens zwei im wesentlichen stabförmige Klemmfinger angeordnet sind, deren distale Enden in Richtung auf den Führungskörper hinweisen. Die Klemmfinger unterstützen die Funktion der besonders geformten Austrittsöffnung, indem sie die Austrittsöffnung zumindest teilweise in Richtung auf den Führungskörper hin verlängern und dadurch die Gefäßprothese zusätzlich führen.

Es wäre grundsätzlich denkbar, daß die Klemmfinger ungeschützt von der Katheterhülle abstehen, allerdings ist es günstig, wenn am Führungskörper Klemmfingeraufnahmen zum Aufnehmen der distalen Enden der Klemmfinger vorgesehen sind. Beim Einführen des Katheters in das Gefäß sind die Enden der Klemmfinger im Führungskörper verborgen, wodurch die Verletzungsgefahr für das Gefäß minimiert wird.

Prinzipiell wären auch in Längsrichtung der Katheterhülle bewegliche Klemmfinger denkbar, besonders vorteilhaft ist es aber, wenn das distale Ende der Klemmfinger in radialer Richtung bewegbar ist. Durch derart bewegbare Klemmfinger läßt sich die teilweise von den Klemmfingern begrenzte Austrittsöffnung praktisch stufenlos bzw. zumindest in Teilbereichen bis zum maximalen Innendurchmesser der Gefäßprothese hin erweitern, wobei jederzeit die Führung der Prothese gewährleistet ist.

Dabei kann vorzugsweise vorgesehen sein, daß die Klemmfinger von einer Symmetrieachse des Gefäßes weg in Richtung auf eine innere Wandung des Gefäßes hin verschwenkbar sind. Die an der Katheterhülle angeordneten Klemmfinger bilden auf diese Weise eine Art Trichter und führen die Prothese während des Übergangs von der durch die Austrittsöffnung durchtretenden Form bis zum geöffneten Zustand.

Der Katheter kann im Innern hohl und glatt sein. Besonders vorteilhaft ist es jedoch, wenn sich im Innern der Katheterhülle eine Innenführung für die Gefäßprothese erstreckt, wenn die Innenführung im Innern der in dem Einführkatheter befindlichen Gefäßprothese angeordnet ist und wenn die Innenführung mindestens im Bereich der Austrittsöffnung eine der Austrittsöffnung geometrisch ähnliche Querschnittsform aufweist. Demnach umgibt die Prothese die Innenführung und wird aufgrund der Formung der Innenführung derart verformt, daß der Austritt aus der Katheterhülle besonders einfach wird. Außerdem verhindert die Innenführung bereits ein Verdrehen der Prothese innerhalb der Katheterhülle. Selbst wenn diese verdreht wäre, würde die Prothese durch die Innenführung wieder in die gewünschte Form gebracht werden.

Dabei kann es günstig sein, wenn sich die Innenführung im wesentlichen über die gesamte Länge der Katheterhülle erstreckt. Auf diese Weise wird die Prothese während des gesamten Einsetzvorgangs verdrehsicher geführt.

Grundsätzlich wäre es möglich, daß die Austrittsöffnung lediglich konvexe Krümmungsbereiche aufwiese. Besonders vorteilhaft ist es jedoch, wenn die Außenkontur der Austrittsöffnung konkave und konvexe Krümmungsbereiche aufweist. Auf diese Weise bildet sich eine strukturierte Kontur der Austrittsöffnung, die gegenüber einer rein konvexen Krümmung deutlich verbesserte Führungseigenschaften aufweist, da die konkaven Krümmungsbereiche weiter in die Austrittsöffnung hineinragen als die konvexen.

Dabei ist es besonders günstig, wenn der Klemmfinger an einen konkaven Krümmungsbereich der Außenkontur der Austrittsöffnung angrenzend angeordnet ist. Die konkaven Krümmungsbereiche ragen zwangsläufig weiter in die Austrittsöffnung hinein als die konvexen Krümmungsbereiche. Durch die Anordnung der Klemmfinger werden diese hineinragenden Bereiche über die Austrittsöffnung hinaus in Richtung auf den Führungskörper hin verlängert und die Form der Prothese kann bis zu deren maximal geöffnetem Zustand optimal geführt werden.

Um jegliche Bildung von Kanten beim Übergang von der Austrittsöffnung zu den Klemmfingern zu vermeiden, kann vorgesehen sein, daß der Klemmfinger mit einem Teil seiner Oberfläche den konkaven Krümmungsbereich bildet. Ein nahtloser Übergang zwischen Austrittsöffnung und Klemmfinger ist dadurch möglich.

Die Oberfläche der Katheterhülle kann völlig glatt und unstrukturiert sein. Besonders vorteilhaft ist es jedoch, wenn die Katheterhülle eine innere hohle Querschnittsfläche aufweist, die mindestens in einem Abschnitt der Katheterhülle der Austrittsöffnung geometrisch ähnlich ist. Eine derartige Ausformung der Innenseite der Katheterhülle optimiert das Austreten der Prothese aus der Austrittsöffnung dadurch, daß die Prothese vor dem Austreten bereits in die erforderliche Form gebracht wird. Ein Verdrehen ist praktisch unmöglich, so daß ein unbeabsichtigter Verschluß der Prothese beim Einsetzen wirkungsvoll verhindert wird.

Es kann vorgesehen sein, daß die Vorrichtung zum Falten durch von einer Innenwandung der Katheterhülle abstehende Leitvorsprünge gebildet wird. Solche Vorsprünge, beispielsweise derart, daß deren radiale Erstreckung nach innen gerichtet kontinuierlich zunimmt in Richtung auf die Austrittsöffnung hin, gestatten eine Faltung während des Zurückziehens der Katheterhülle. In einem Arbeitsgang kann dadurch das Falten und das Einsetzen mit einer maximalen Sicherheit gegen unbeabsichtigtes Verdrehen realisiert werden.

Der Außendurchmesser der Katheterhülle kann zwar größer sein als der Innendurchmesser der Gefäßprothese, besonders vorteilhaft ist es jedoch, wenn der Außendurchmesser der Katheterhülle kleiner als der Innendurchmesser der geöffneten Gefäßprothese ist. Dadurch kann die Katheterhülle innerhalb einer eingesetzten Gefäßprothese reibungsfrei verschoben werden.

Bei einer weiteren bevorzugten Ausführungsform der Erfindung kann vorgesehen sein, daß der Außendurchmesser der Katheterhülle dem Außendurchmesser des Führungskörpers entspricht. Dadurch läßt sich eine vollständig glatte Außenhaut des Katheters realisieren, denn kantige Übergänge und Vorsprünge an der Außenhülle des Katheters würden die Verletzungsgefahr beim Einführen des Katheters in das Gefäß erhöhen.

Grundsätzlich könnte der Katheter eine starre Hülle aufweisen, günstig ist es jedoch, wenn die Katheterhülle durch einen elastischen Schlauch gebildet wird. Ein elastischer Schlauch läßt sich innerhalb eines Gefäßsystems auch durch gekrümmte Gefäßbahnen einführen, ohne letztere zu verletzen.

Vorteilhafterweise kann auch vorgesehen sein, daß der Haltekörper in einer Einführstellung des Einführkatheters radial teilweise von den Klemmfingern begrenzt wird. Der innerhalb der Prothese befindliche Haltekörper kann dann bei einer Ausdehnung in radialer Richtung die Prothese gegen die Klemmfinger drücken und somit zusätzlich zu einer Führung der Prothese beitragen. Außerdem können die Klemmfinger durch den Haltekörper radial nach außen aufgespreizt werden.

Außerdem kann vorteilhafterweise vorgesehen sein, daß der Haltekörper in einer Einführstellung des Einführkatheters radial mindestens teilweise von dem Führungskörper umgeben wird. Dadurch wird der Haltekörper beim Einführen des Katheters durch den Führungskörper geschützt.

Zum Einsetzen der Gefäßprothese kann das proximale Ende des Katheters von einem Operateur erfaßt werden. Vorteilhafterweise kann hierfür vorgesehen sein, daß am proximalen Ende der Katheterhülle ein diese umgebender Griffbereich vorgesehen ist. Auf diese Weise kann der Operateur den Katheter sicher führen und halten.

Für eine optimale Führung des Katheters durch den Operateur ist es von Vorteil, wenn er den Katheter stets in der Nähe einer Einführstelle des Katheters in den Körper des Patienten ergreifen kann, insbesondere dann, wenn die Katheterhülle biegbar ist. Zu diesem Zweck ist es vorteilhaft, wenn der Griffbereich relativ zur Katheterhülle verschiebbar ist.

Bei einer bevorzugten Ausführungsform der Erfindung kann vorgesehen sein, daß am Griffbereich in radialer Richtung nach innen abstehende Vorsprünge angeordnet sind. Derartige Vorsprünge erhöhen die Reibung zwischen Griffbereich und Katheterhülle, so daß der Operateur den Katheter jederzeit sicher und genau führen kann.

Beim Zurückziehen des Katheters während des Einsetzens der Prothese kann ein sehr langer Abschnitt der Katheterhülle aus der Einführstelle am Körper des Patienten hervorragen. Daher kann es vorteilhaft sein, wenn die Vorsprünge durch in Richtung auf das distale Ende hin angeschliffene Messerklingen gebildet werden. Diese ermöglichen eine Spaltung der Katheterhülle, so daß das Innere der Katheterhülle besonders leicht zugänglich ist und die Katheterhülle besonders leicht aus dem Gefäß wieder entfernt werden kann. Ferner wird dadurch die maximale Länge des Führungsstabes, der eigentlich doppelt so lang sein müßte wie die Katheterhülle, auf eine Länge reduziert, die etwa der Länge der Katheterhülle entspricht.

Besonders vorteilhaft kann es sein, wenn die Austrittsöffnung im wesentlichen eine Hundeknochenform aufweist.

Bei einer anderen Ausführungsform kann vorgesehen sein, daß die Austrittsöffnung im wesentlichen eine Pilzform aufweist.

Günstig kann es auch sein, wenn die Austrittsöffnung im wesentlichen eine Kleeblattform aufweist.

Dabei kann es für eine optimierte Führung vorteilhaft sein, wenn die Kleeblattform unterschiedlich große Blätter umfaßt.

Andererseits kann es auch günstig sein, wenn die Austrittsöffnung im wesentlichen eine Kreuzform mit abgerundeten Kanten aufweist.

Bei einer weiteren bevorzugten Ausführungsform kann jedoch auch vorgesehen sein, daß die Austrittsöffnung im wesentlichen eine sichel- oder halbmondförmige Form aufweist.

Die nachfolgende Beschreibung bevorzugter Ausführungsformen der Erfindung dient im Zusammenhang mit der Zeichnung der näheren Erläuterung. Es zeigen:
- Fig. 1:: einen Längsschnitt durch das distale Ende eines Einführkatheters beim Einführen in ein Gefäß;
- Fig. 2:: einen Längsschnitt durch ein Gefäß und das Ende des Katheters mit inflatiertem Haltekörper;
- Fig. 3:: einen Querschnitt längs Linie 3-3 aus Figur 2 eines ersten Ausführungsbeispiels einer Austrittsöffnung;
- Fig. 4:: eine Schnittansicht analog Figur 3 durch ein zweites Ausführungsbeispiel einer Austrittsöffnung;
- Fig. 5:: eine Schnittansicht ähnlich Figur 1 durch einen Katheter mit einer alternativen Anordnung des Haltekörpers;
- Fig. 6:: einen Längsschnitt ähnlich Figur 1, jedoch mit an der Katheterhülle angeordneten Klemmfingern;
- Fig. 7:: einen Querschnitt längs Linie 7-7 aus Figur 6.
- Fig. 7a:: einen Querschnitt längs Linie 7-7 mit inflatiertem Haltekörper;
- Fig. 7b:: einen Querschnitt längs Linie 7b-7b aus Figur 6;
- Fig. 8:: einen Längsschnitt durch das proximale Ende eines Katheters;
- Fig. 9a bis 9c:: alternative Ausführungsformen der Austrittsöffnung;
- Fig. 10:: einen Längsschnitt ähnlich Figur 2, jedoch mit einem selbstexpandierenden Stent als Haltekörper; und
- Fig. 11:: einen Längsschnitt ähnlich Figur 10, jedoch mit einem an der Gefäßprothese angeordneten inflatierbaren Haltekörper.

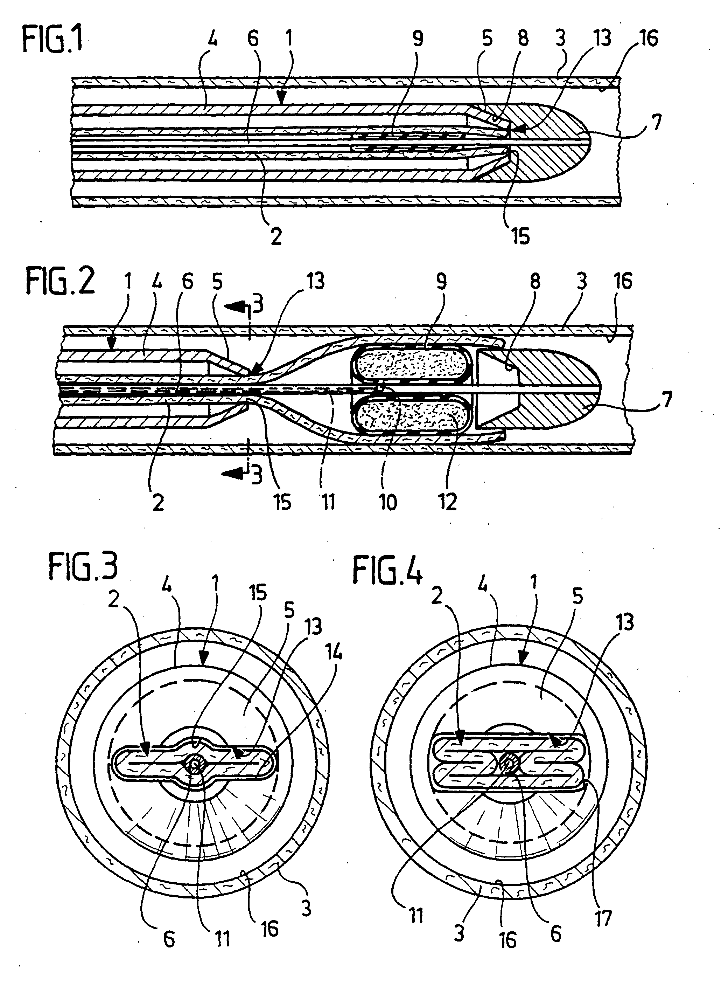

In den Figuren 1 bis 11 sind verschiedene Varianten eines insgesamt mit dem Bezugszeichen 1 versehenen Einführkatheters zum Einführen einer Gefäßprothese 2 in ein Gefäß 3 dargestellt. Ähnliche Teile sind der Übersichtlichkeit wegen mit denselben Bezugszeichen versehen.

Der Einführkatheter 1 besteht im wesentlichen aus einer schlauchförmigen Katheterhülle 4, die nahezu über ihre gesamte Länge einen konstanten Querschnitt aufweist. An ihrem distalen Ende ist die Katheterhülle 4 konisch verjüngt, so daß dieses Ende die Form eines Außenkonus 5 aufweist. Die Wandstärke der Katheterhülle 4 ist im wesentlichen über die gesamte Länge der Katheterhülle 4 konstant.

Entlang ihrer Symmetrieachse wird die Katheterhülle 4 von einem Führungsstab 6 durchsetzt, an dessen distalem Ende ein Leitkörper 7 angeordnet ist. Das distale Ende des Leitkörpers 7 ist halbkugelförmig abgerundet, das proximale Ende ist in Form eines zum Außenkonus 5 komplementären Innenkonus 8 geformt, so daß das distale Ende der Katheterhülle 4 in das proximale Ende des Leitkörpers 7 formschlüssig einsteckbar ist. Im Querschnitt entspricht der Durchmesser des Leitkörpers 7 dem der Katheterhülle 4, so daß bei in den Leitkörper 7 eingesteckter Katheterhülle 4 eine vollständig glatte Außenhaut des Katheters 1 gebildet wird.

Der Führungsstab 6 wird auf einem Abschnitt, der an den Leitkörper 7 angrenzt und in etwa dessen Länge aufweist, ringförmig von einem Ballon 9 umgeben, der über eine Querbohrung 10 mit einem im Führungsstab 6 verlaufenden Kanal 11 in Fluidverbindung steht, wobei der Kanal 11 vom proximalen Ende des Führungsstabes 6 aus mit einem Fluid 12 beschickt werden kann, so daß der Ballon 9 von einem deflatierten Zustand, bei dem er im wesentlichen vollständig am Führungsstab 6 anliegt, wie in Figur 1 zu sehen, in einen inflatierten Zustand übergeht, in dem er eine im wesentlichen zylindrische Form einnimmt und dessen Außendurchmesser dann im wesentlichen einem Innendurchmesser des Gefäßes 3 entspricht, wie in Figur 2 zu sehen ist.

Schließlich ist innerhalb der Katheterhülle 4 die Gefäßprothese 2 derart angeordnet, daß sie sowohl den Führungsstab 6 als auch den Ballon 9 vollständig umgibt.

Der Katheter 1 weist am distalen Ende der Katheterhülle 4 eine Austrittsöffnung 13 auf, die im wesentlichen durch einen länglichen Schlitz 14 gebildet wird, der wiederum eine Breite aufweist, die im wesentlichen dem Doppelten der Wandstärke der Gefäßprothese 2 entspricht. In ihrem Mittelbereich sind an der Austrittsöffnung 13 einander gegenüberliegende Ausbuchtungen 15 angeordnet, so daß die einfach gefaltete und den Führungsstab 6 umgebende Gefäßprothese 2 durch den Schlitz 14 durchzogen werden kann.

Zum Einsetzen der Gefäßprothese 2 wird der Katheter 1 in das Gefäß 3 so weit eingeführt, bis die Gefäßprothese 2 eine beispielsweise brüchige Stelle des Gefäßes 3 vollständig überdeckt. Die Katheterhülle 4 wird relativ zur Gefäßprothese 2 und dem Führungsstab 6 so weit zurückgezogen, bis der Ballon 9, der sich ursprünglich innerhalb der Katheterhülle 4 befindet, vollständig aus der Katheterhülle ausgetreten ist. Anschließend wird durch den Kanal 11 das Fluid 12, beispielsweise eine isotonische Kochsalzlösung, in den Ballon 9 geleitet, bis dieser so weit inflatiert ist, daß er die Gefäßprothese 2 mit seiner äußeren Umfangsfläche gegen eine Innenwandung 16 des Gefäßes 3 drückt und dort hält. Die Katheterhülle 4 wird danach schrittweise zurückgezogen, bis die Gefäßprothese 2 vollständig freigegeben ist und an der Innenwandung 16 anliegt. Abschließend wird der Ballon 9 durch Ablassen des Fluids 12 deflatiert und der Führungsstab 6 zusammen mit dem Leitkörper 7 zurückgezogen. Gegebenenfalls kann die Gefäßprothese 2 mittels einer nicht dargestellten Anastomose an dem Gefäß 3 fixiert werden, beispielsweise durch Klammern oder Nähen.

In Figur 4 ist ein zweites Beispiel einer möglichen Austrittsöffnung 13 dargestellt, die die Form eines Rechtecks 17 aufweist. Eine kürzere Seite und damit die Breite des Rechtecks 17 entspricht etwa viermal der Wandstärke der Gefäßprothese 2. Die Querschnittsfläche des Rechtecks 17 ist so bemessen, daß die doppelt gefaltete Gefäßprothese 2 den Führungsstab 6 umgibt und insgesamt einen minimalen Querschnitt einnimmt. Nur in diesem gefalteten Zustand kann die Gefäßprothese 2 durch die Austrittsöffnung 13 in Form des Rechtecks 17 durchtreten. Vor dem Einsetzen des Einführkatheters 1 kann die Gefäßprothese 2 bereits in diesem gefalteten Zustand ins Innere der Katheterhülle 4 gebracht werden, oder aber erst vor dem Austreten durch nicht dargestellte Leitvorsprünge im Innern der Katheterhülle 4, die der Austrittsöffnung 13 zugewandt sind, gefaltet werden.

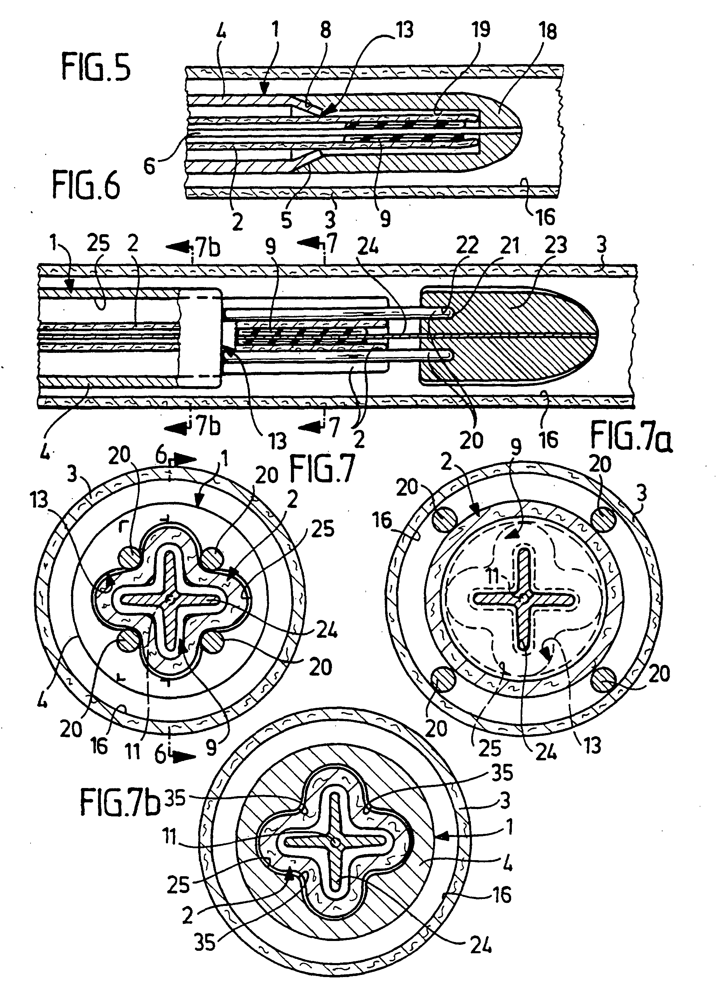

Eine alternative Ausgestaltung eines Leitkörpers 18 ist in Figur 5 dargestellt. Er unterscheidet sich vom Leitkörper 7 in Figur 1 dadurch, daß von seinem proximalen Ende aus eine den Leitkörper 18 nahezu vollständig durchsetzende Sacklochbohrung 19 angebracht ist, die wiederum vom Führungsstab 6, der mit der Spitze des Leitkörpers 18 verbunden ist, durchsetzt wird. Der den Führungsstab 6 umgebende Ballon 9 befindet sich in der Einführstellung des Katheters 1 vollständig innerhalb der Sacklochbohrung 19 und wird daher auf seiner gesamten Länge vom Leitkörper 18 umgeben und dadurch geschützt.

Das Einführen einer Gefäßprothese 2 in ein Gefäß 3 mit einem Einführkatheter 1, wie er in Figur 5 dargestellt ist, läuft nach dem bereits oben beschriebenen Prinzip ab. Zu berücksichtigen ist allerdings, daß der Leitkörper 18 relativ zum Führungsstab 6 beweglich ausgebildet ist, so daß vor dem Inflatieren des Ballons 9 der Leitkörper 18 in distaler Richtung von dem Ballon 9 wegbewegt wird, so daß dieser die Gefäßprothese 2 an die Innenwandung des Gefäßes 3 andrücken kann, bevor die Gefäßprothese 2 durch Rückziehen der Katheterhülle 4 innerhalb des Gefäßes 3 positioniert wird.

In den Figuren 6 und 7 ist eine abgewandelte Ausführungsform des Einführkatheters 1 dargestellt. Am distalen Ende der Katheterhülle 4 sind vier Leitstäbe 20 in konkaven Krümmungsbereichen der im wesentlichen kleeblattförmigen Austrittsöffnung 13 in Längsrichtung der Katheterhülle 4 abstehend angeordnet. Leitstabenden 21 sind in der Einführstellung des Einführkatheters 1 in korrespondierende sacklochartige Leitstabaufnahmen 22 eines geschoßförmigen Leitkörpers 23 eingesteckt.

Im Innern der Katheterhülle 4 ist eine im Querschnitt kreuzförmige Innenführung 24 entlang der Symmetrieachse der Katheterhülle 4 verlaufend angeordnet und erstreckt sich über das distale Ende der Katheterhülle 4 hinaus bis etwa zum proximalen Ende des Leitkörpers 23. Diese Innenführung 24 ist im Bereich der Leitstäbe 20 von einem Ballon 9 umgeben, der in seinem deflatierten Zustand, wie in Figur 7 dargestellt, ebenfalls eine Kreuzform einnimmt. Die Innenführung 24 und damit auch der Ballon 9 im Bereich der Leitstäbe 20 sind von der Gefäßprothese 2 umgeben, die an die Innenführung 24 anliegend ebenfalls eine Kreuzform einnimmt. Im Innern ist die Katheterhülle 4 komplementär, aber beabstandet zur Innenführung 24 geformt, so daß zwischen der Innenführung 24 und einer inneren Katheterwand 25 ein Spalt verbleibt, der von der Gefäßprothese 2 ausgefüllt wird.

Zum Einsetzen der Gefäßprothese 2 wird der Einführkatheter 1 in seiner Einführstellung mit den in den Leitkörper 23 eingesteckten Leitstäben 20 in das Gefäß 3 eingeführt. Sobald die endgültige Position für die Gefäßprothese 2 erreicht ist, wird die Katheterhülle 4 in proximaler Richtung zurückgezogen, bis die Leitstabenden 21 aus den Leitstabaufnahmen 22 ausgetreten sind. Der Ballon 9 wird dann durch Einleiten eines Fluids 12 inflatiert, wodurch er im Querschnitt eine im wesentlichen kreisförmige Form annimmt und dadurch die Leitstäbe 20 von der Symmetrieachse der Katheterhülle 4 weg in Richtung auf die Innenwandung 16 des Gefäßes 3 verschwenkt. Auf diese Weise wird der Übergang der in einer Kleeblattform aus der Austrittsöffnung 13 austretenden Gefäßprothese 2 kontinuierlich durch die Leitstäbe 20 geführt, bis die Gefäßprothese 2 einen im wesentlichen kreisförmigen Querschnitt einnimmt und an der Innenwandung 16 des Gefäßes 3 anliegt. Die im Querschnitt aufgeweitete Stellung der Leitstäbe 20 ist in Figur 7a zu sehen. Die Leitstäbe 20 stellen demnach eine teilweise Verlängerung der Austrittsöffnung 13 zur Führung der Gefäßprothese 2 während des Einsetzens dar.

An seinem proximalen Ende weist der Einführkatheter 1 einen hülsenförmigen Griff 26 auf, dessen Innendurchmesser etwas größer ist als der Außendurchmesser der Katheterhülle 4. Ein proximales Griffende 27 des Griffs 26 ist verschlossen, aber mit einer zentralen Bohrung 28 versehen, deren Innendurchmesser dem Außendurchmesser der Katheterhülle 4 entspricht, so daß der Griff 26 spielfrei auf der Katheterhülle 4 gleiten kann. Von einer inneren Griffwandung 29 ragen zwei Messer 30 radial nach innen vor, die jeweils eine in Richtung auf das distale Ende der Katheterhülle 4 abgeschrägte Schneide 31 aufweisen. Die Messer 30 ragen radial gerade so weit vor, daß sie die Katheterhülle 4 vollständig durchdringen, die innerhalb der Katheterhülle 4 gelagerte Gefäßprothese 2 jedoch nicht berühren und somit auch nicht verletzen.

Die besondere Ausgestaltung des Griffs 26 dient dazu, die Katheterhülle 4 beim Zurückziehen zu spalten und dadurch das Entfernen derselben aus dem Gefäß 3 zu erleichtern. Insbesondere ist dadurch der Führungsstab 6 bzw. die Innenführung 24 jederzeit auch noch bei zurückgezogener Katheterhülle 4 zugänglich und vom Operateur bedienbar.

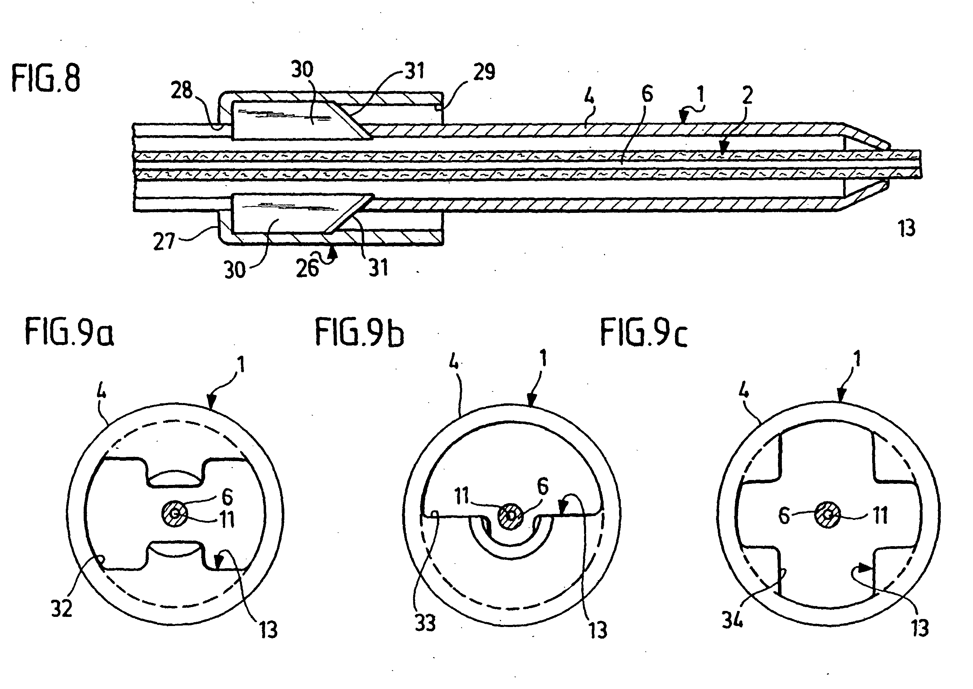

In den Figuren 9a, 9b und 9c sind schematisch weitere Formen von Austrittsöffnungen 13 des Einführkatheters 1 dargestellt. Figur 9a zeigt eine Hundeknochenform 32, Figur 9b eine Pilzform 33 und Figur 9c eine Kleeblattform 34, bei der einander gegenüberliegende blattähnliche Ausbuchtungen identisch ausgebildet sind, ansonsten jedoch paarweise eine unterschiedliche Größe aufweisen.

In Verbindung mit den Austrittsöffnungen 32 bis 34 können ebenfalls Leitstäbe 20 vorgesehen sein, die jeweils in konkaven Krümmungsbereichen der Austrittsöffnungen **32** bis 34 angeordnet sind. Darüber hinaus sind geometrisch ähnlich geformte Innenführungen 24 in Verbindung mit den Austrittsöffnungen 32 bis 34 möglich. Selbstverständlich ist eine entsprechende komplementäre Formung der inneren Katheterwand 25 möglich. Gerade durch diese komplementäre Formgebung von Katheterwand 25 und Innenführung 24, die quasi eine Faltvorrichtung bilden, ist das Falten der Gefäßprothese 2 möglich, wenn letztere durch Zurückziehen der Katheterhülle 4 durch eine der Austrittsöffnungen 32 bis 34 durchtritt. Radial nach innen vorstehende Bereiche der Katheterwand 25 bilden auf diese Weise Leitvorsprünge 35 zum Falten der Gefäßprothese 2.

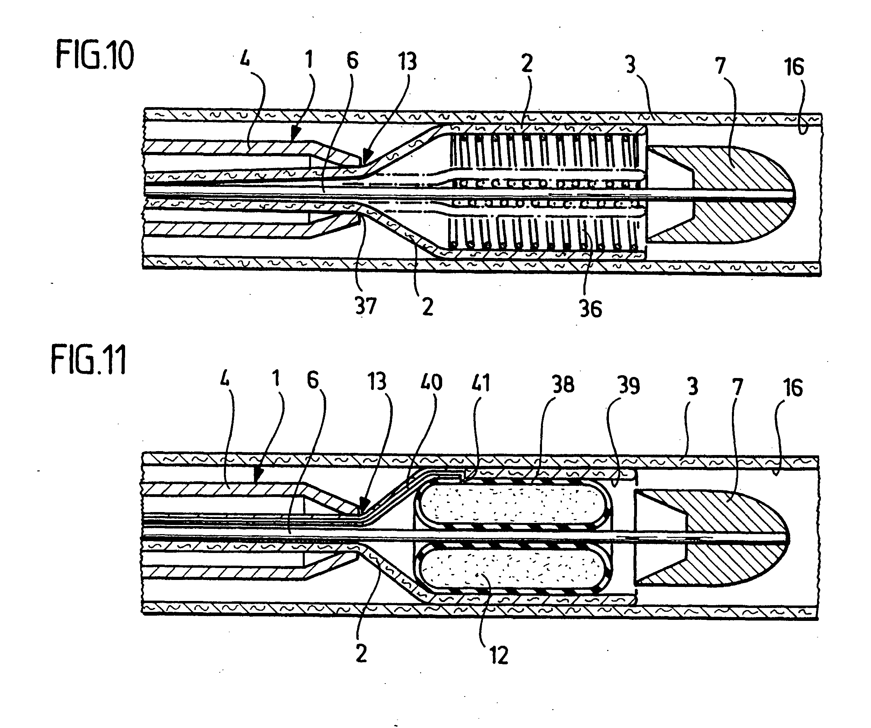

Figur 10 zeigt einen Längsschnitt ähnlich Figur 2, wobei jedoch anstelle des Ballons 9 ein selbstexpandierender Stent 36 vorgesehen ist. Dieser kann beispielsweise aus Metall oder einem speziellen Memory-Metall gebildet sein, das beim Einsetzen des Einführkatheters 1 in das Gefäß 3 im wesentlichen an dem Führungsstab 6 anliegt, sich zum Halten der Gefäßprothese 2 und gleichzeitigen Andrücken derselben an die Innenwandung 16 des Gefäßes 3 aufgrund thermischer Aktivierung verformt und dadurch einen größeren Durchmesser einnimmt. Der Stent 36 kann wahlweise auch vollständig in die Gefäßprothese 2 integriert oder an einer äußeren Umfangswandung derselben angeordnet sein.

Allerdings zeigt Figur 10 die Anordnung des Stents 36 nur rein schematisch, so daß dieser bei einer Ausführungsform am Führungsstab 6, bei einer anderen Ausführungsform aber auch an der Gefäßprothese 2 angeordnet sein kann.

Ein weiterer Unterschied zu dem in der Figur 2 dargestellten Einführkatheter 1 besteht darin, daß die Austrittsöffnung 13 eine halbmondförmige Querschnittsfläche 37 aufweist. Es sind jedoch auch beliebige andere Formen der Querschnittsfläche 37 denkbar, beispielsweise auch die im Zusammenhang mit den Figuren 9a, 9b und 9c beschriebenen.

Figur 11 zeigt einen Längsschnitt ähnlich Figur 2. Als Haltekörper ist ein ringförmiger Ballon 38 vorgesehen, der an einer inneren Prothesenwand 39 der Gefäßprothese 2 angeordnet ist. Über einen von proximal nach distal in der Wand der Gefäßprothese 2 verlaufenden Kanal 40, der über eine radial nach innen gerichtete Querverbindung 41 in Fluidverbindung mit dem Ballon 38 steht, ist letzterer durch Einströmenlassen eines Fluids 12 inflatierbar. Der mit dem Führungsstab 6 nicht fest verbundene Ballon 38 stützt sich beim Inflatieren und in seinem inflatierten Zustand lediglich am Führungsstab 6 ab, so daß die Gefäßprothese 2 an der Innenwandung 16 des Gefäßes 3 gehalten wird.

Die Anordnung des Haltekörpers ist in Figur 11 lediglich schematisch dargestellt. So bildet die der inneren Prothesenwand 39 abgewandte Oberfläche des Ballons 38 selbst einen Teil der inneren Prothesenwand. Alternativ wäre es denkbar, den Ballon 38 in die Wand der Gefäßprothese 2 zu integrieren, so daß sich beim Inflatieren des Ballons 38 die Wandstärke der Gefäßprothese 2 ändert, nämlich zunimmt, und dadurch die Gefäßprothese 2 an dem Gefäß 3 hält, ohne daß Teile des Ballons 38 nach dem Deflatieren desselben ins Innere des Gefäßes 3 vorstehen und einen Fluß eines im Gefäß 3 fließenden Mediums behindern.

Denkbar wäre es auch, einen Stent 36 und einen Ballon 9 bzw. 38 gemeinsam vorzusehen, beispielsweise derart, daß der Stent 36 den Ballon 38 umgibt oder auch umgekehrt, wobei das Inflatieren des Ballons 9 bzw. 38 gleichzeitig den Stent 36 inflatiert, wobei letzterer auch selbstexpandierbar sein kann.

Das Einsetzen der Gefäßprothese 2 in das Gefäß 3 wird bei den in Verbindung mit den Figuren 10 und 11 beschriebenen Einführkathetern 1 im wesentlichen genauso durchgeführt, wie es bereits oben, insbesondere im Zusammenhang mit den Figuren 1 und 2, beschrieben wurde.

## Patentansprüche

1. Einführkatheter (1), der eine stützstrukturlose Gefäßprothese trägt, zum Einführen der Gefäßprothese (2) in ein Gefäß (3), mit einer langgestreckten, im wesentlichen rohrförmigen Katheterhülle (4), die ein proximales und ein distales Ende aufweist, wobei an dem distalen Ende eine Austrittsöffnung für die Gefäßprothese (2) vorgesehen ist, wobei die von der Austrittsöffnung (13, 14, 17, 32, 33, 34) definierte Querschnittsfläche kleiner als die Querschnittsfläche der in das Gefäß (3) eingebrachten Gefäßprothese (2) im geöffneten ungedehnten Zustand ist, **dadurch gekennzeichnet, daß** die Austrittsöffnung (13, 14, 17, 32, 33, 34) eine im Querschnitt von einer Kreisform abweichende Form aufweist und daß sich im Innern der Katheterhülle (4) eine Vorrichtung (24, 25) zum Falten der Gefäßprothese (2) in einem an die Austrittsöffnung (13, 14, 17, 32, 33, 34) angrenzenden Abschnitt erstreckt, wodurch die Gefäßprothese beim Durchtritt durch die Austrittsöffnung (13, 14, 17, 32, 33, 34) eine dafür notwendige Querschnittsform einnimmt.

2. Einführkatheter nach Anspruch 1, **dadurch gekennzeichnet, daß** die Außenkontur der Austrittsöffnung (13, 14, 17, 32, 33, 34) eine Umfangslänge aufweist, die mindestens dem Umfang der in das Gefäß (3) eingebrachten Gefäßprothese (2) entspricht.

3. Einführkatheter nach Anspruch 1, **dadurch gekennzeichnet, daß** die Außenkontur der Austrittsöffnung (13, 14, 17) eine Umfangslänge aufweist, die kleiner als der äußere Umfang der in das Gefäß (3) eingebrachten Gefäßprothese (2) ist.

4. Einführkatheter nach Anspruch 3, **dadurch gekennzeichnet, daß** die Austrittsöffnung eine Rechteckform (14, 17) aufweist, die eine kürzere Seitenkante mit einer Breite aufweist, die mindestens einem geradzahligen Vielfachen einer Wandstärke der Gefäßprothese (2) entspricht.

5. Einführkatheter nach einem der Ansprüche 3 oder 4, **dadurch gekennzeichnet, daß** die Querschnittsfläche der Austrittsöffnung (14, 17) im wesentlichen identisch zu der Querschnittsfläche einer auf ihren kleinstmöglichen Querschnitt gefalteten und durch die Austrittsfläche (14, 17) auszubringenden Gefäßprothese (2) ist.

6. Einführkatheter nach einem der voranstehenden Ansprüche, **dadurch gekennzeichnet, daß** das distale Ende der Katheterhülle einen Führungskörper (7, 18, 23) umfaßt.

7. Einführkatheter nach Anspruch 6, **dadurch gekennzeichnet, daß** der Führungskörper (7, 18, 23) relativ zur Katheterhülle (4) in einer von der Katheterhülle (4) vorgegebenen Längsrichtung verschiebbar ist.

8. Einführkatheter nach einem der Ansprüche 6 oder 7, **dadurch gekennzeichnet, daß** der Führungskörper (7, 18, 23) am Ende eines die Katheterhülle (4) durchsetzenden Führungsstabes (6, 24) angeordnet ist.

9. Einführkatheter nach Anspruch 8, **dadurch gekennzeichnet, daß** am Führungsstab (6, 24) im Bereich des Führungskörpers (7, 18, 23) mindestens ein in seinem äußeren Umfang in radialer Richtung veränderbarer Haltekörper (9) angeordnet ist.

10. Einführkatheter nach Anspruch 8, **dadurch gekennzeichnet, daß** an der Gefäßprothese (2) im Bereich des Führungskörpers (7, 18, 23) mindestens ein in seinem äußeren Umfang in radialer Richtung veränderbarer Haltekörper (36, 38) angeordnet ist.

11. Einführkatheter nach Anspruch 10, **dadurch gekennzeichnet, daß** der Haltekörper eine selbstexpandierbare Gefäßstütze (36) umfaßt.

12. Einführkatheter nach Anspruch 11, **dadurch gekennzeichnet, daß** die Gefäßstütze (36) durch ein Metall gebildet wird.

13. Einführkatheter nach einem der Ansprüche 9 bis 11, **dadurch gekennzeichnet, daß** der Haltekörper (9) mittels eines Fluids (12) inflatierbar ist.

14. Einführkatheter nach Anspruch 13, **dadurch gekennzeichnet, daß** der Haltekörper einen Ballon (9, 38) umfaßt.

15. Einführkatheter nach Anspruch 13 oder 14, **dadurch gekennzeichnet, daß** im Innern des Führungsstabes (6, 24) eine mit dem Innern des Haltekörpers (9, 38) in Fluidverbindung stehende Fluidleitung (11) verläuft.

16. Einführkatheter nach einem der Ansprüche 9 bis 15, **dadurch gekennzeichnet, daß** der Haltekörper (9) den Führungsstab (6, 24) ringförmig umgibt.

17. Einführkatheter nach einem der Ansprüche 6 bis 16, **dadurch gekennzeichnet, daß** das distale Ende (5) der Katheterhülle (4) und das proximale Ende (8) des Führungskörpers (7, 18) eine zueinander komplementäre Form aufweisen.

18. Einführkatheter nach Anspruch 17, **dadurch gekennzeichnet, daß** das distale Ende der Katheterhülle (4) durch einen Außenkonus (5) und das proximale Ende des Führungskörpers (7, 18) durch einen Innenkonus (8) gebildet werden.

19. Einführkatheter nach einem der Ansprüche 6 bis 18, **dadurch gekennzeichnet, daß** das distale Ende des Führungskörpers (7, 18, 23) eine abgerundete Spitze aufweist.

20. Einführkatheter nach einem der Ansprüche 6 bis 19, **dadurch gekennzeichnet, daß** das distale Ende der Katheterhülle (4) durch den Führungskörper (7, 18, 23) verschließbar ist.

21. Einführkatheter nach einem der Ansprüche 6 bis 20, **dadurch gekennzeichnet, daß** an dem distalen Ende der Katheterhülle (4) mindestens zwei im wesentlichen stabförmige Klemmfinger (20) angeordnet sind, deren distale Enden in Richtung auf den Führungskörper (23) hin weisen.

22. Einführkatheter nach Anspruch 21, **dadurch gekennzeichnet, daß** am Führungskörper (23) Klemmfingeraufnahmen (22) zum Aufnehmen der distalen Enden (21) der Klemmfinger (20) vorgesehen sind.

23. Einführkatheter nach einem der Ansprüche 21 oder 22, **dadurch gekennzeichnet, daß** das distale Ende (21) der Klemmfinger (20) in radialer Richtung bewegbar ist.

24. Einführkatheter nach Anspruch 23, **dadurch gekennzeichnet, daß** die Klemmfinger (20) von einer Symmetrieachse **der Katheterhülle (4) weg** verschwenkbar sind.

25. Einführkatheter nach einem der voranstehenden Ansprüche, **dadurch gekennzeichnet, daß** sich im Innern der Katheterhülle (4) eine Innenführung (24) für die Gefäßprothese (2) erstreckt, daß die Innenführung (24) im Innern der in dem Einführkatheter (1) befindlichen Gefäßprothese (2) angeordnet ist und daß die Innenführung (24) mindestens im Bereich der Austrittsöffnung (13, 14, 17, 32, 33, 34) eine der Austrittsöffnung (13, 14, 17, 32, 33, 34) geometrisch ähnliche Querschnittsform aufweist.

26. Einführkatheter nach Anspruch 25, **dadurch gekennzeichnet, daß** sich die Innenführung (24) im wesentlichen über die gesamte Länge der Katheterhülle (4) erstreckt.

27. Einführkatheter nach einem der voranstehenden Ansprüche, **dadurch gekennzeichnet, daß** die Außenkontur der Austrittsöffnung (13, 32, 33, 34) konkave und konvexe Krümmungsbereiche aufweist.

28. Einführkatheter nach Anspruch 27 in Kombination mit Anspruch 21, **dadurch gekennzeichnet, daß** die Klemmfinger (20) an einen konkaven Krümmungsbereich der Außenkontur der Austrittsöffnung (13, 32, 33, 34) angrenzend angeordnet sind.

29. Einführkatheter nach Anspruch 28, **dadurch gekennzeichnet, daß** die Klemmfinger (20) mit einem Teil seiner Oberfläche den konkaven Krümmungsbereich bildet.

30. Einführkatheter nach einem der voranstehenden Ansprüche, **dadurch gekennzeichnet, daß** die Katheterhülle (4) eine innere hohle Querschnittsfläche aufweist, die mindestens in einem Abschnitt der Katheterhülle (4) der Austrittsöffnung (13, 14, 17, 32, 33, 34) geometrisch ähnlich ist.

31. Einführkatheter nach einem der voranstehenden Ansprüche, **dadurch gekennzeichnet, daß** die Vorrichtung zum Falten durch von einer Innenwandung (25) der Katheterhülle (4) abstehende Leitvorsprünge (35) gebildet wird.

32. Einführkatheter nach einem der voranstehenden Ansprüche, **dadurch gekennzeichnet, daß** der Außendurchmesser der Katheterhülle (4) kleiner als der Innendurchmesser der geöffneten Gefäßprothese (2) ist.

33. Einführkatheter nach einem der Ansprüche 6 bis 32, **dadurch gekennzeichnet, daß** der Außendurchmesser der Katheterhülle (4) dem Außendurchmesser des Führungskörpers (7, 18, 23) entspricht.

34. Einführkatheter nach einem der voranstehenden Ansprüche, **dadurch gekennzeichnet, daß** die Katheterhülle durch einen elastischen Schlauch (4) gebildet wird.

35. Einführkatheter nach einem der Ansprüche 19 bis 34, **dadurch gekennzeichnet, daß** der Haltekörper (9) in einer Einführstellung des Einführkatheters (1) radial teilweise von den Klemmfingern (20) begrenzt wird.

36. Einführkatheter nach einem der Ansprüche 21 bis 35, **dadurch gekennzeichnet, daß** der Haltekörper (9) in einer Einführstellung des Einführkatheters (1) radial mindestens teilweise von dem Führungskörper (18) umgeben wird.

37. Einführkatheter nach einem der voranstehenden Ansprüche, **dadurch gekennzeichnet, daß** am proximalen Ende der Katheterhülle (4) ein diese umgebender Griffbereich (26) vorgesehen ist.

38. Einführkatheter nach Anspruch 37, **dadurch gekennzeichnet, daß** der Griffbereich (26) relativ zur Katheterhülle (4) verschiebbar ist.

39. Einführkatheter nach einem der Ansprüche 37 oder 38, **dadurch gekennzeichnet, daß** am Griffbereich (26) in radialer Richtung nach innen abstehende Vorsprünge (30) angeordnet sind.

40. Einführkatheter nach Anspruch 39, **dadurch gekennzeichnet, daß** die Vorsprünge durch in Richtung auf das distale Ende hin angeschliffene Messerklingen (30) gebildet werden.

41. Einführkatheter nach einem der Ansprüche 32 bis 40 in Kombination mit einem der Ansprüche 1 oder 2 und 7 bis 30, **dadurch gekennzeichnet, daß** die Austrittsöffnung im wesentlichen eine Hundeknochenform (32) aufweist.

42. Einführkatheter nach einem der Ansprüche 32 bis 40 in Kombination mit einem der Ansprüche 1 oder 2 und 7 bis 30, **dadurch gekennzeichnet, daß** die Austrittsöffnung im wesentlichen eine Pilzform (33) aufweist.

43. Einführkatheter nach einem der Ansprüche 32 bis 40 in Kombination mit einem der Ansprüche 1 oder 2 und 7 bis 30, **dadurch gekennzeichnet, daß** die Austrittsöffnung im wesentlichen eine Kleeblattform (13, 34) aufweist.

44. Einführkatheter nach Anspruch 43, **dadurch gekennzeichnet, daß** die Kleeblattform (34) unterschiedlich große Blätter umfaßt.

45. Einführkatheter nach einem der Ansprüche 32 bis 40 in Kombination mit einem der Ansprüche 1 oder 2 und 7 bis 30, **dadurch gekennzeichnet, daß** die Austrittsöffnung im wesentlichen eine Kreuzform (13) mit abgerundeten Kanten aufweist.

46. Einführkatheter nach einem der Ansprüche 32 bis 40 in Kombination mit einem der Ansprüche 1 oder 2 und 7 bis 30, **dadurch gekennzeichnet, daß** die Austrittsöffnung im wesentlichen eine sichel- oder halbmondförmige Form (37) aufweist.

## Claims

1. An insertion catheter (1), which carries a vascular prosthesis without a supporting structure, for inserting the vascular prosthesis (2) into a vessel (3), with an elongated, substantially tubular catheter sheath (4), which has a proximal and a distal end, an outlet for the vascular prosthesis (2) being provided at the distal end, the cross-sectional area defined by the outlet (13, 14, 17, 32, 33, 34) being smaller than the cross-sectional area of the vascular prosthesis (2) introduced into the vessel (3) in the opened, unexpanded state, **characterised in that** the outlet (13, 14, 17, 32, 33, 34) has a shape differing from a circular shape in cross-section, and **in that** a device (24, 25) for folding the vascular prosthesis (2) extends in the interior of the catheter sheath (4) in a portion adjoining the outlet (13, 14, 17, 32, 33, 34), as a result of which the vascular prosthesis on passing through the outlet (13, 14, 17, 32, 33, 34) assumes a cross-sectional shape required therefor.

2. An insertion catheter according to Claim 1, **characterised in that** the outer contour of the outlet (13, 14, 17, 32, 33, 34) has a circumferential length which corresponds at least to the circumference of the vascular prosthesis (2) introduced into the vessel (3).

3. An insertion catheter according to Claim 1, **characterised in that** the outer contour of the outlet (13, 14, 17) has a circumferential length which is smaller than the outer circumference of the vascular prosthesis (2) introduced into the vessel (3).

4. An insertion catheter according to Claim 3, **characterised in that** the outlet has a rectangular shape (14, 17) which has a shorter side edge with a width which corresponds at least to an even-numbered multiple of a wall thickness of the vascular prosthesis (2).

5. An insertion catheter according to one of Claims 3 or 4, **characterised in that** the cross-sectional area of the outlet (14, 17) is substantially identical to the cross-sectional area of a vascular prosthesis (2) which is folded to its smallest possible cross-section and is to be brought out through the outlet area (14, 17).

6. An insertion catheter according to one of the preceding Claims, **characterised in that** the distal end of the catheter sheath comprises a guide body (7, 18, 23).

7. An insertion catheter according to Claim 6, **characterised in that** the guide body (7, 18, 23) is displaceable relative to the catheter sheath (4) in a longitudinal direction prescribed by the catheter sheath (4).

8. An insertion catheter according to one of Claims 6 or 7, **characterised in that** the guide body (7, 18, 23) is arranged at the end of a guide rod (6, 24) extending through the catheter sheath (4).

9. An insertion catheter according to Claim 8, **characterised in that** at least one holding body (9) variable in the radial direction in its outer circumference is arranged on the guide rod (6, 24) in the region of the guide body (7, 18, 23).

10. An insertion catheter according to Claim 8, **characterised in that** at least one holding body (36, 38) variable in the radial direction in its outer circumference is arranged on the vascular prosthesis (2) in the region of the guide body (7, 18, 23).

11. An insertion catheter according to Claim 10, **characterised in that** the holding body comprises a self-expandable vascular support (36).

12. An insertion catheter according to Claim 11, **characterised in that** the vascular support (36) is formed by a metal.

13. An insertion catheter according to one of Claims 9 to 11, **characterised in that** the holding body (9) is inflatable by means of a fluid (12).

14. An insertion catheter according to Claim 13, **characterised in that** the holding body comprises a balloon (9, 38).

15. An insertion catheter according to Claim 13 or 14, **characterised in that** a fluid line (11) in fluid connection with the interior of the holding body (9, 38) extends in the interior of the guide rod (6, 24).

16. An insertion catheter according to one of Claims 9 to 15, **characterised in that** the holding body (9) surrounds the guide rod (6, 24) in an annular shape.

17. An insertion catheter according to one of Claims 6 to 16, **characterised in that** the distal end (5) of the catheter sheath (4) and the proximal end (8) of the guide body (7, 18) have a mutually complementary shape.

18. An insertion catheter according to Claim 17, **characterised in that** the distal end of the catheter sheath (4) is formed by an outer cone (5) and the proximal end of the guide body (7, 18) by an inner cone (8).

19. An insertion catheter according to one of Claims 6 to 18, **characterised in that** the distal end of the guide body (7, 18, 23) has a rounded tip.

20. An insertion catheter according to one of Claims 6 to 19, **characterised in that** the distal end of the catheter sheath (4) is closable by means of the guide body (7, 18, 23).

21. An insertion catheter according to one of Claims 6 to 20, **characterised in that** at least two substantially rod-shaped clamping fingers (20), the distal ends of which are directed towards the guide body (23), are arranged at the distal end of the catheter sheath (4).

22. An insertion catheter according to Claim 21, **characterised in that** clamping finger receptacles (22) for receiving the distal ends (21) of the clamping fingers (20) are provided on the guide body (23).

23. An insertion catheter according to one of Claims 21 or 22, **characterised in that** the distal end (21) of the clamping fingers (20) is movable in the radial direction.

24. An insertion catheter according to Claim 23, **characterised in that** the clamping fingers (20) are pivotable away from an axis of symmetry of the catheter sheath (4).

25. An insertion catheter according to one of the preceding Claims, **characterised in that** an interior guide (24) for the vascular prosthesis (2) extends in the interior of the catheter sheath (4), **in that** the interior guide (24) is arranged in the interior of the vascular prosthesis (2) located in the insertion catheter (1), and **in that** the interior guide (24) has a cross-sectional shape geometrically similar to the outlet (13, 14, 17, 32, 33, 34) at least in the region of the outlet (13, 14, 17, 32, 33, 34).

26. An insertion catheter according to Claim 25, **characterised in that** the interior guide (24) extends substantially over the entire length of the catheter sheath (4).

27. An insertion catheter according to one of the preceding Claims, **characterised in that** the outer contour of the outlet (13, 32, 33, 34) has concave and convex curvature regions.

28. An insertion catheter according to Claim 27 in combination with Claim 21, **characterised in that** the clamping fingers (20) are arranged adjacent to a concave curvature region of the outer contour of the outlet (13, 32, 33, 34).

29. An insertion catheter according to Claim 28, **characterised in that** the clamping fingers (20) form with part of their surface the concave curvature region.

30. An insertion catheter according to one of the preceding Claims, **characterised in that** the catheter sheath (4) has an internal hollow cross-sectional area which is geometrically similar to the outlet (13, 14, 17, 32, 33, 34) at least in one portion of the catheter sheath (4).

31. An insertion catheter according to one of the preceding Claims, **characterised in that** the device for folding is formed by guide projections (35) protruding from an interior wall (25) of the catheter sheath (4).

32. An insertion catheter according to one of the preceding Claims, **characterised in that** the outer diameter of the catheter sheath (4) is smaller than the inside diameter of the opened vascular prosthesis (2).

33. An insertion catheter according to one of Claims 6 to 32, **characterised in that** the outer diameter of the catheter sheath (4) corresponds to the outer diameter of the guide body (7, 18, 23).

34. An insertion catheter according to one of the preceding Claims, **characterised in that** the catheter sheath is formed by a flexible tube (4).

35. An insertion catheter according to one of Claims 19 to 34, **characterised in that** the holding body (9) is radially partially delimited by the clamping fingers (20) in an inserting position of the insertion catheter (1).

36. An insertion catheter according to one of Claims 21 to 35, **characterised in that** the holding body (9) is radially at least partially surrounded by the guide body (18) in an inserting position of the insertion catheter (1).

37. An insertion catheter according to one of the preceding Claims, **characterised in that** at the proximal end of the catheter sheath (4) a grip region (26) surrounding the said sheath is provided.

38. An insertion catheter according to Claim 37, **characterised in that** the grip region (26) is displaceable relative to the catheter sheath (4).

39. An insertion catheter according to one of Claims 37 or 38, **characterised in that** projections (30) protruding inwardly in the radial direction are arranged on the grip region (26).

40. An insertion catheter according to Claim 39, **characterised in that** the projections are formed by knife blades (30) sharpened in the direction towards the distal end.

41. An insertion catheter according to one of Claims 32 to 40 in combination with one of Claims 1 or 2 and 7 to 30, **characterised in that** the outlet has substantially a dog's bone shape (32).

42. An insertion catheter according to one of Claims 32 to 40 in combination with one of Claims 1 or 2 and 7 to 30, **characterised in that** the outlet has substantially a mushroom shape (33).

43. An insertion catheter according to one of Claims 32 to 40 in combination with one of Claims 1 or 2 and 7 to 30, **characterised in that** the outlet has substantially a cloverleaf shape (13, 34).

44. An insertion catheter according to Claim 43, **characterised in that** the cloverleaf shape (34) comprises leaves of different sizes.

45. An insertion catheter according to one of Claims 32 to 40 in combination with one of Claims 1 or 2 and 7 to 30, **characterised in that** the outlet has substantially a cross shape (13) with rounded edges.

46. An insertion catheter according to one of Claims 32 to 40 in combination with one of Claims 1 or 2 and 7 to 30, **characterised in that** the outlet has substantially a crescent or half-moon shape (37).

## Revendications

1. Cathéter d'insertion (1), qui porte une prothèse vasculaire sans structure de soutien, destiné à introduire la prothèse vasculaire (2) dans un vaisseau (3), et comprenant une enveloppe de cathéter (4) allongée, sensiblement de forme tubulaire, qui présente une extrémité proximale et distale, une ouverture de sortie pour la prothèse vasculaire (2) étant prévue à l'extrémité distale, et la surface de section transversale définie par l'ouverture de sortie (13, 14, 17, 32, 33, 34) étant plus petite que la surface de section transversale de la prothèse vasculaire (2) insérée dans le vaisseau, à l'état ouvert non dilaté, **caractérisé en ce que** l'ouverture de sortie (13, 14, 17, 32, 33, 34) présente en section transversale, une forme s'écartant d'une forme circulaire, et **en ce que** dans l'intérieur de l'enveloppe de cathéter (4) s'étend un dispositif (24, 25) destiné à plier la prothèse vasculaire (2) dans une zone adjacente de l'ouverture de sortie (13, 14, 17, 32, 33, 34), ce qui fait que la prothèse vasculaire (2), lors de son passage à travers l'ouverture de sortie (13, 14, 17, 32, 33, 34), prend une forme de section transversale nécessaire à cet effet.

2. Cathéter d'insertion selon la revendication 1, **caractérisé en ce que** le contour extérieur de l'ouverture de sortie (13, 14, 17, 32, 33, 34) présente une périphérie ou périmètre d'une longueur qui correspond au moins à la périphérie ou au périmètre de la prothèse vasculaire (2) insérée dans le vaisseau (3).

3. Cathéter d'insertion selon la revendication 1, **caractérisé en ce que** le contour extérieur de l'ouverture de sortie (13, 14, 17) présente une périphérie ou un périmètre d'une longueur qui est inférieure à la périphérie extérieure ou périmètre extérieur de la prothèse vasculaire (2) insérée dans le vaisseau (3).

4. Cathéter d'insertion selon la revendication 3, **caractérisé en ce que** l'ouverture de sortie présente une forme rectangulaire (14, 17) dont le petit côté a une largeur qui correspond au moins à un multiple entier de l'épaisseur de paroi de la prothèse vasculaire (2).

5. Cathéter d'insertion selon l'une des revendications 3 ou 4, **caractérisé en ce que** la surface de section transversale de l'ouverture de sortie (14, 17) est sensiblement identique à la surface de section transversale d'une prothèse vasculaire (2) pliée à sa section transversale la plus petite possible et devant être extraite à travers la surface de sortie (14, 17).

6. Cathéter d'insertion selon l'une des revendications précédentes, **caractérisé en ce que** l'extrémité distale de l'enveloppe de cathéter comprend un corps de guidage (7, 18, 23).

7. Cathéter d'insertion selon la revendication 6, **caractérisé en ce que** le corps de guidage (7, 18, 23) peut coulisser par rapport à l'enveloppe de cathéter (4) dans une direction longitudinale prédéfinie par l'enveloppe de cathéter (4).

8. Cathéter d'insertion selon l'une des revendications 6 ou 7, **caractérisé en ce que** le corps de guidage (7, 18, 23) est disposé à l'extrémité d'une tige de guidage (6, 24) traversant l'enveloppe de cathéter (4).

9. Cathéter d'insertion selon la revendication 8, **caractérisé en ce que** sur la tige de guidage (6, 24), dans la zone du corps de guidage (7, 18, 23), est disposé au moins un corps de maintien (9) pouvant être modifié quant à sa périphérie extérieure, dans la direction radiale.

10. Cathéter d'insertion selon la revendication 8, **caractérisé en ce que** sur la prothèse vasculaire (2), dans la zone du corps de guidage (7, 18, 23), est disposé au moins un corps de maintien (36, 38) pouvant être modifié quant à sa périphérie extérieure, dans la direction radiale.

11. Cathéter d'insertion selon la revendication 10, **caractérisé en ce que** le corps de maintien comprend un élément de soutien de vaisseau (36) auto-expansible.

12. Cathéter d'insertion selon la revendication 11, **caractérisé en ce que** l'élément de soutien de vaisseau (36) et formé par un métal.

13. Cathéter d'insertion selon l'une des revendications 9 à 11, **caractérisé en ce que** le corps de maintien (9) peut être gonflé au moyen d'un fluide (12).

14. Cathéter d'insertion selon la revendication 13, **caractérisé en ce que** le corps de maintien comprend un ballonnet (9, 38).

15. Cathéter d'insertion selon la revendication 13 ou 14, **caractérisé en ce qu'**à l'intérieur de la tige de guidage (6, 24) s'étend une conduite de fluide (11) se trouvant en liaison d'écoulement de fluide avec l'intérieur du corps de maintien (9, 38).

16. Cathéter d'insertion selon l'une des revendications 9 à 15, **caractérisé en ce que** le corps de maintien (9) entoure la tige de guidage (6, 24) de manière annulaire.

17. Cathéter d'insertion selon l'une des revendications 6 à 16, **caractérisé en ce que** l'extrémité distale (5) de l'enveloppe de cathéter (4) et l'extrémité proximale (8) du corps de guidage (7, 18) présentent une forme mutuellement complémentaire.

18. Cathéter d'insertion selon la revendication 17, **caractérisé en ce que** l'extrémité distale de l'enveloppe de cathéter (4) est formée par un cône extérieur (5) et l'extrémité proximale du corps de guidage (7, 18) est formée par un cône intérieur (8).

19. Cathéter d'insertion selon l'une des revendications 6 à 18, **caractérisé en ce que** l'extrémité distale du corps de guidage (7, 18, 23) présente une pointe arrondie.

20. Cathéter d'insertion selon l'une des revendications 6 à 19, **caractérisé en ce que** l'extrémité distale de l'enveloppe de cathéter (4) peut être obturée par le corps de guidage (7, 18, 23).

21. Cathéter d'insertion selon l'une des revendications 6 à 20, **caractérisé en ce qu'**à l'extrémité distale de l'enveloppe de cathéter (4) sont disposés au moins deux doigts de serrage (20) sensiblement en forme de tige, dont les extrémités distales sont dirigées en direction du corps de guidage (23).

22. Cathéter d'insertion selon la revendication 21, **caractérisé en ce que** sur le corps de guidage (23) sont prévus des logements de doigts de serrage (22) destinés à recevoir les extrémités distales (21) des doigts de serrage (20).

23. Cathéter d'insertion selon l'une des revendications 21 ou 22, **caractérisé en ce que** l'extrémité distale (21) des doigts de serrage (20) est mobile dans la direction radiale.

24. Cathéter d'insertion selon la revendication 23, **caractérisé en ce que** les doigts de serrage (20) peuvent pivoter de façon à s'éloigner de l'axe de symétrie de l'enveloppe de cathéter (4).

25. Cathéter d'insertion selon l'une des revendications précédentes, **caractérisé en ce qu'**à l'intérieur de l'enveloppe de cathéter (4) s'étend un guidage intérieur (24) pour la prothèse vasculaire (2), **en ce que** le guidage intérieur (24) est disposé à l'intérieur de la prothèse vasculaire (2) se trouvant dans le cathéter d'insertion (1), et **en ce que** le guidage intérieur (24) présente, au moins dans la zone de l'ouverture de sortie (13, 14, 17, 32, 33, 34), une forme de section transversale similaire sur le plan géométrique, à celle de l'ouverture de sortie (13, 14, 17, 32, 33, 34).

26. Cathéter d'insertion selon la revendication 25, **caractérisé en ce que** le guidage intérieur (24) s'étend sensiblement sur la totalité de la longueur de l'enveloppe de cathéter (4).

27. Cathéter d'insertion selon l'une des revendications précédentes, **caractérisé en ce que** le contour extérieur de l'ouverture de sortie (13, 32, 33, 34) présente des zones de courbure concaves et convexes.

28. Cathéter d'insertion selon la revendication 27, en combinaison avec la revendication 21, **caractérisé en ce que** les doigts de serrage (20) sont disposés de manière adjacente à une zone de courbure concave du contour extérieur de l'ouverture de sortie (13, 32, 33, 34).

29. Cathéter d'insertion selon la revendication 28, **caractérisé en ce que** les doigts de serrage (20) forment, avec une partie de leur surface, la zone de courbure concave.

30. Cathéter d'insertion selon l'une des revendications précédentes, **caractérisé en ce que** l'enveloppe de cathéter (4) présente une surface de section transversale intérieure creuse, qui au moins dans une zone de l'enveloppe de cathéter (4), est similaire sur le plan géométrique, à l'ouverture de sortie (13, 14, 17, 32, 33, 34).

31. Cathéter d'insertion selon l'une des revendications précédentes, **caractérisé en ce que** le dispositif pour le pliage est formé par des protubérances de guidage (35) faisant saillie de la paroi intérieure (25) de l'enveloppe de cathéter (4).

32. Cathéter d'insertion selon l'une des revendications précédentes, **caractérisé en ce que** le diamètre extérieur de l'enveloppe de cathéter (4) est plus petit que le diamètre intérieur de la prothèse vasculaire (2) ouverte.

33. Cathéter d'insertion selon l'une des revendications 6 à 32, **caractérisé en ce que** le diamètre extérieur de l'enveloppe de cathéter (4) correspond au diamètre extérieur du corps de guidage (7, 18, 23).

34. Cathéter d'insertion selon l'une des revendications précédentes, **caractérisé en ce que** l'enveloppe de cathéter est formée par un tuyau ou une tubulure élastique (4).

35. Cathéter d'insertion selon l'une des revendications 19 à 34, **caractérisé en ce que** le corps de maintien (9), dans une position d'insertion du cathéter d'insertion (1), est délimité radialement, au moins partiellement, par les doigts de serrage (20).

36. Cathéter d'insertion selon l'une des revendications 21 à 35, **caractérisé en ce que** le corps de maintien (9), dans une position d'insertion du cathéter d'insertion (1), est entouré radialement, au moins partiellement, par le corps de guidage (18).

37. Cathéter d'insertion selon l'une des revendications précédentes, **caractérisé en ce qu'**à l'extrémité proximale de l'enveloppe de cathéter (4), est prévue une zone de préhension (26) entourant ladite enveloppe de cathéter.

38. Cathéter d'insertion selon la revendication 37, **caractérisé en ce que** la zone de préhension (26) peut coulisser par rapport à l'enveloppe de cathéter (4).

39. Cathéter d'insertion selon l'une des revendications 37 ou 38, **caractérisé en ce que** sur la zone de préhension (26) sont disposées des protubérances (30) faisant saillie vers l'intérieur, dans la direction radiale.

40. Cathéter d'insertion selon la revendication 39, **caractérisé en ce que** les protubérances sont formées par des lames de coupe (30) affûtées en direction de l'extrémité distale.

41. Cathéter d'insertion selon l'une des revendications 32 à 40, en combinaison avec l'une des revendications 1 ou 2 et 7 à 30, **caractérisé en ce que** l'ouverture de sortie présente sensiblement une forme d'os de chien (32).

42. Cathéter d'insertion selon l'une des revendications 32 à 40, en combinaison avec l'une des revendications 1 ou 2 et 7 à 30, **caractérisé en ce que** l'ouverture de sortie présente sensiblement une forme de champignon (33).

43. Cathéter d'insertion selon l'une des revendications 32 à 40, en combinaison avec l'une des revendications 1 ou 2 et 7 à 30, **caractérisé en ce que** l'ouverture de sortie présente sensiblement une forme de feuille de trèfle (13, 34).

44. Cathéter d'insertion selon la revendication 43, **caractérisé en ce que** la forme de feuille de trèfle (34) comprend des feuilles de différentes grandeurs.

45. Cathéter d'insertion selon l'une des revendications 32 à 40, en combinaison avec l'une des revendications 1 ou 2 et 7 à 30, **caractérisé en ce que** l'ouverture de sortie présente sensiblement une forme de croix (13) avec des arêtes arrondies.

46. Cathéter d'insertion selon l'une des revendications 32 à 40, en combinaison avec l'une des revendications 1 ou 2 et 7 à 30, **caractérisé en ce que** l'ouverture de sortie présente sensiblement une forme de faucille ou de demi-lune (37).
